(19) Europäisches Patentamt

European Patent Office

Office européen des brevets

(11) EP 1 214 972 B1

(12) **EUROPEAN PATENT SPECIFICATION**

(45) Date of publication and mention
of the grant of the patent:
**04.06.2003 Bulletin 2003/23**

(51) Int Cl.⁷: **B01J 23/80**, C07D 315/00, C07D 307/33

(21) Application number: **00311192.9**

(22) Date of filing: **14.12.2000**

(54) **Method for preparing lactone**

Verfahren für die Herstellung von Lacton

Méthode de préparation de lactone

(84) Designated Contracting States:
**BE DK FR GB IT**

(43) Date of publication of application:
**19.06.2002 Bulletin 2002/25**

(73) Proprietor: **Dairen Chemical Corporation Taipei (TW)**

(72) Inventors:
• **Chen, Shien-Chang**
**Taipei, Taiwan (TW)**
• **Hsu, Liang-An**
**Ta She Industrial Zone Kaohsiung, Taiwan (TW)**
• **Lin, Fu-Shen**
**Ta She Industrial Zone Kaohsiung, Taiwan (TW)**
• **Tsai, Cheng-Lin**
**Ta She Industrial Zone Kaohsiung, Taiwan (TW)**

(74) Representative: **Jones, Helen Marjorie Meredith**
**Gill Jennings & Every,**
**Broadgate House,**
**7 Eldon Street**
**London EC2M 7LH (GB)**

(56) References cited:
**EP-A- 0 271 139      EP-A- 0 281 417**
**US-A- 4 683 218      US-A- 5 072 009**
**US-A- 5 955 620**

**Description**

**Field of the Invention**

The present invention relates to a method for preparing a lactone, which comprises a dehydrocyclization reaction of a diol under a gas phase in the presence of the aforementioned catalyst after activating said catalyst.

**Background of the Invention**

Lactone, such as γ-butyrolactone, may be used as a herbicide, used in the pharmaceutical composition, or used for preparing an intermediate; this intermediate is used to prepare pyrrolidone (such as N-methylpyrrolidone, 2-pyrrolidone and N-vinylpyrrolidone), piperidine, phenylbutyric acid and thiobutyric acid. Thus, developing an economic method for preparing γ-butyrolactone is a common industrial requirement.

Previously, γ-butyrolactone was commonly produced by a hydrogenation reaction with maleic anhydride or maleic acid ester under liquid phase or gas phase. However, this is an undesirable industrial process because the process needs a lot of hydrogen gas and its catalyst has a short lifetime.

Recently, γ-butyrolactone was produced by a dehydrocyclization reaction with 1,4-butanediol, where a hydrogen by-product was generated as a raw material and as fuel. This method for preparing γ-butyrolactone through the dehydrocyclization reaction of 1,4-butanediol is disclosed in JP-A-58-013575 (1983), wherein the dehydrocyclization reaction is carried out under liquid phase using a platinum/lead catalyst; however, the activity of said catalyst is low and the selectivity of γ-butyrolactone is also low. The method disclosed in JP-A-61-246173 (1986) describes that γ-butyrolactone is obtained by passing 1,4-butanediol vapor through a copper/chromium/zinc catalyst; however, this method may generate many tetrahydrofuran and butanol by-products, and the selectivity and yield of γ-butyrolactone is usually low. The method disclosed in JP-A-03-232874 describes that γ-butyrolactone is produced by passing 1,4-butanediol vapor through a copper/chromium/manganese or barium catalyst; the method disclosed in US-A-5,110,954 describes that γ-butyrolactone is obtained by adding 1,4-butanediol into the solution of copper/chromium catalyst; and the method disclosed in JP-A-02-255668 describes that γ-butyrolactone is produced by passing 1,4-butanediol vapor through a copper/zinc/alkali metal catalyst. However, the activity of these catalysts may decay quickly, and the conversion of 1,4-butylene glycol may become lower after reacting over a long period. Therefore, it is undesirable as an industrial process.

The present inventors have deeply studied the above defects of the traditional technique and found more effective catalyst for preparing a lactone in the catalyst supporting a cupric compound, a zinc compound and at least one alkaline earth metal compound. The process for preparing lactone through the dehydrocyclization reaction of diol using the above catalyst under gas phase may increase the activity and lifetime of catalyst, while the selectivity may be up to 99 mol% or more; therefore the beneficial economic effect of industrial processes may be substantially increased. We have hereby accomplished the present invention.

EP-A-0271139 discloses a process for converting monobasic alcohols to carboxylic acids by reaction in the gas phase with water and hydrogen in the presence of an activated catalyst comprising a cupric compound, a zinc compound and optionally an alkali metal or alkaline earth metal compound on a support. In EP-A-0281417 similar catalysts are used to catalyse a liquid phase reaction between an alcohol and a primary or secondary amine to form an alkylated amine.

**Summary of the Invention**

The present invention relates to a method for preparing a lactone, which comprises a dehydrocyclization reaction of a diol under a gas phase in the presence of a catalyst which comprises a cupric compound, a zinc compound and at least one alkaline earth metal compound on a support after activating said catalyst by reducing it at a temperature ranging from 180 to 250°C for 6 to 20 hours with hydrogen gas. The catalyst for preparing lactone of the present invention is quite economic because of its high activity, long lifetime and high selectivity of products.

**Detailed Description of the Invention**

The catalyst used for preparing a lactone in the present invention is prepared by supporting a cupric compound, a zinc compound and at least one alkaline earth metal compound on a support. The suitable materials for the support used in the catalyst of the present invention are silica, alumina, or their mixture, more preferably the mixture of silica and alumina.

In the catalyst used for preparing lactone in the invention, the cupric compound may be selected from various cupric salts, of which examples are copper (II) nitrate ($Cu(NO_3)_2$ $3H_2O$), copper (II) carbonate ($Cu_2(OH)_2CO_3$), copper

(II) acetate (Cu(CH$_3$COO)$_2$), copper (II) chloride (CuCl$_2$ 2H$_2$O), copper (II) hydroxide (Cu(OH)$_2$), copper(II) phosphate (Cu$_3$(PO$_4$)$_2$3H$_2$O) and copper(ll) sulfate (CuSO$_4$ 5H$_2$O). The zinc compound used in the catalyst of the present invention may be selected from various zinc salts, of which examples are zinc nitrate (Zn(NO$_3$)$_2$ 6H$_2$O), zinc carbonate (ZnCO$_3$), zinc acetate (Zn(CH$_3$COO)$_2$ 2H$_2$O), zinc chloride (ZnCl$_2$), zinc hydroxide (Zn(OH)$_2$) and zinc sulfate (ZnSO$_4$ 7H$_2$O). The alkaline earth metal compound used In the catalyst of the present invention is at least one selected from metal compounds of beryllium, magnesium, calcium, strontium and barium, and more preferably at least one selected from a metal compound of magnesium, calcium and barium, which is, for instance, a their carbonate, hydroxide, silicate or phosphate.

The catalyst used for preparing a lactone in the invention is prepared according to the following method. The support is immersed in the above aqueous cupric salt and zinc salt solutions, and the value of pH is adjusted between 8 and 11 using ammonia water, while the hydroxides of . copper and zinc are precipitated on the support. The precipitate is washed with water and dried. The precipitate is immersed in the above aqueous salts solution selected from one or two alkaline earth metal compound(s) of magnesium, calcium and barium, and then calcined for 3 to 5 hours at 400 to 500°C. If it is necessary, a mold-aid agent such as graphite may be added, and a predeterminate shape is molded by a molding machine. In such resultant catalyst, each metal component exists in the form of oxide. Therefore, before the dehydrogenation reaction of diol, the catalyst must be reduced and be activated at a temperature ranging.from 180 to 250°C for 6 to 20 hours with hydrogen gas wherein the ratio of hydrogen gas to nitrogen gas starts between 1 : 20 and 1:10 by volume, then gradually adjusts to all hydrogen gas.

In the catalyst used for preparing lactone in the invention the ratio of copper (II) oxide to zinc oxide is usually in the range 6 : 1 to 1 : 2 by weight, preferably in the range 5:1 to 1:1. When any one of the alkaline earth metal compounds selected from a group consisting of magnesium, calcium and barium is used, its amount is preferably in the range 0.01 to 10 wt%, more preferably 0.05 to 5 wt%, based on the total weight of copper (II) oxide and zinc oxide in terms of oxides. When any two of the alkaline earth metal compounds selected from a group consisting of magnesium, calcium and barium is used, their amounts are preferably in the range 0.5 to 20 wt%, more preferably 1 to 10 wt%, based on the total weight of copper (II) oxide and zinc oxide in terms of oxides. The amount of support is preferably 0.5 to 20 wt%, more preferably 1 to 10 wt%, based on the total weight of copper (II) oxide and zinc oxide in terms of silica.

The example of the lactone used in the method for preparing lactone of the present invention includes, for instance, β-propiolactone, β-butyrolactone, γ-butyrolactone, γ-valerolactone, δ-butyrolactone, γ-caprolactone, ε-caprolactone, δ-hydroxyoctylic acid lactone, δ-hydroxynonylic acid lactone, γ-hydroxydecylic acid lactone, δ-hydroxynonylic acid lactone, etc.

The example of the diol used in the method for preparing lactone of the present invention includes, for instance, 1,3-propylene glycol, 2-methyl-1,3-. propylene glycol, 1,3-butylene glycol, 1,4-butylene glycol, 1,5-pentanediol, 1,4-pentanediol, 1,5-hexanediol, 1,6-hexanediol, 1,7-heptanediol, 1,8-octanediol, 1,9-nonanediol, 1,10-decanediol; etc.

In the dehydrocyclization reaction of the method for preparing lactone such as γ-butyrolactone used in the present invention, the reaction temperature usually ranges from 160 to 280°C, preferably 180 to 250°C. If the reaction temperature is too low, the conversion of 1,4-butylene glycol may be decreased. Although the higher temperature may increase the conversion of 1,4-butylene glycol, the selectivity of γ-butyrolactone may be substantially decreased.

In the dehydrocyclization reaction of the method for preparing lactone such as γ-butyrolactone used in the present invention, the reaction pressure usually ranges from 0 to 10 atm (0 to 10$^6$Pa), preferably from 1 to 5 atm ((1-5) x 10$^5$Pa). However, a higher reaction pressure may easily carry out an undesired reaction, decreasing the yield.

In the dehydrocyclization reaction of the method for preparing lactone such as γ-butyrolactone used in the present invention, the hydrogen gas is needed as a carrier gas. If the hydrogen gas do not exist in the reaction system, the lifetime of catalyst may be shortened. The amount of hydrogen gas must at least maintain the reaction system in gas phase. In generally, the molar ratio of hydrogen gas to 1,4-butylene glycol used in the present invention ranges from (12 to 1): 1, preferably from (8 to 1.5): 1.

In the dehydrocyclization reaction of the method for preparing lactone such as γ-butyrolactone used in the present invention, if the gas hourly space velocity of 1,4-butylene glycol is too low, the retention time of gas in the catalyst bed is also too long so that the product may be decomposed, resulting in the decreasing selectivity of γ-butyrolactone. If the gas hourly space velocity of 1,4-butylene glycol is too high, the retention time of gas in the catalyst bed is also too short so that the conversion of 1,4-butylene glycol decreases. In general, the gas hourly space velocity of 1,4-butylene glycol ranges from 10 to 20,000 hr$^{-1}$, preferably 30 to 9,000 hr$^{-1}$.

In the dehydrocyclization reaction of the method for preparing lactone such as γ-butyrolactone used in the present invention, the catalyst bed may be a fixed bed or a fluid bed.

The present invention will be further described in the following Examples and Comparative Examples. However, the scope of the present invention is not restricted by such Examples.

**Example**

In a given time after the dehydrocyclization reaction, the product was collected by condensation. The component of the efflux from the outlet was analyzed by HP-6890 gas chromatograph. The conversion of diol and the selectivity of lactone were calculated according to the following equation (1) and (2), and the yield of lactone is also obtained:

$$\text{The Conversion of Diol (\%) =}$$

$$\frac{\text{Moles of Fed-in-Diol - Moles of Fed-out-Diol}}{\text{Moles of Fed-in-Diol}} \times 100 \qquad (1)$$

$$\text{The Selectivity of Lactone (\%) =}$$

$$\frac{\text{Moles of Lactone Product}}{\text{Moles of Fed-in-Diol - Moles of Fed-out-Diol}} \times 100 \qquad (2)$$

**Reference Example 1**

A commercially available copper-chromium catalyst (30 ml) (wherein copper oxide was 42 wt%, chromium oxide was 28 wt%, and a diameter was 5 mm) was packed in the stainless steel reactor having an inside diameter of 23.5 mm. After the temperature was elevated to 150 with nitrogen gas, the mixed gas of 10 vol% hydrogen gas was passed into the reactor, then the catalytic reduction reaction was initiated. The temperature and the concentration of hydrogen gas were gradually elevated until the reduction temperature of catalyst was 200°C and the concentration of hydrogen gas was 100 vol%. When the temperature of catalyst bed was confirmed to be the same as that of th heating equipment, the reduction reaction was terminated.
Subsequently, the temperature of the reactorwas elevated to 210°C. 1,4-butylene glycol was pumped into the reactor using a quantitative pump, and the gas hourly space velocity of 1,4-butylene glycol was maintained at 4500 hr$^{-1}$. After the dehydrogenation reaction was carried out at a hydrogen gas/1,4-butylene glycol ratio of 5 mole: 1 mole, the product was collected and analyzed. The results are shown in Table 1.

**Reference Example 2**

The same steps as in Reference Example 1 were repeated, but a commercially available copper-zinc catalyst (G-66) was used, wherein the component was 60 wt% of copper oxide and 30 wt% of zinc oxide. The results are shown in Table 1.

**Reference Example 3**

The same steps as in Reference Example 1 were repeated, but a copper/chromium/zinc catalyst which was prepared by the method of JP-A-61-246173 (1986) was used, wherein the component was 35 wt% of copper oxide, 4.5 wt% of zinc oxide and 60 wt% of chromium oxide. The results are shown in Table 1.

**Reference Example 4**

The same steps as in Reference Example 1 were repeated, but a commercially available copper-zinc catalyst (G-66) which was immersed in 0.5 wt% of aqueous sodium hydroxide solution and then dried was used, wherein the component was 60 wt% of copper oxide, 30 wt% of zinc oxide and 0.12 wt% of sodium hydroxide. The results are shown in Table 1.

Table 1

| Reference Example | 1 | 2 | 3 | 4 |
|---|---|---|---|---|
| Reaction Temperature (°C) | 210 | 210 | 210 | 210 |
| Reaction Pressure (atm) ($10^5$Pa) | 1 | 1 | 1 | 1 |
| Gas Hourly Space Velocity of 1,4-Butylene Glycol (hr$^{-1}$) | 4500 | 4500 | 4500 | 4500 |

Table 1   (continued)

| Reference Example | 1 | 2 | 3 | 4 |
|---|---|---|---|---|
| Molar Ratio of Hydrogen Gas to 1,4-Butylene Glycol | 5/1 | 5/1 | 5/1 | 5/1 |
| Conversion of 1,4-Butylene Glycol (mol%) | 72.50 | 91.50 | 81.30 | 97.20 |
| Selectivity of γ-Butyrolactone (mol%) | 88.30 | 92.50 | 90.50 | 95.30 |
| Yield of γ-Butyrolactone (mol%) | 64.02 | 84.64 | 73.82 | 92.63 |

## Comparative Example 1

60wt% of aqueous copper nitrate solution (350 g) was slowly poured into 40 wt% of aqueous zinc nitrate solution (220 g) and silica powder (10 g) wherein the BET surface area was 185 $m^2/g$ (said surface area was measured according to the Brunner-Emmett-Teller Method), and the mixture was stirred vigorously. Subsequently, 25 wt% of ammonia waterwas added to maintain the pH value of the mixed aqueous solution at 10 while the mixture was still stirred. The precipitate was filtered and separated, washed with water, and placed into a oven to dry at 100°C for 12 hours, after which time a catalyst precursor was obtained. This catalyst precursor was moved to a tubular high-temperature furnace heated to 450°C, and calcined for 4 hours. The component of this catalyst was at a copper oxide/zinc oxide ratio of 3 : 1.

1.0 wt% of graphite was added to the above catalyst, then that catalyst was extruded to a round granular catalyst having a diameter of 5 mm. The same steps as in Reference Example 1 were repeated with this catalyst (30ml). The results are shown in Table 2.

## Comparative Example 2 to 7

The same steps as in Example 1 were repeated, but the ratio by weight of copper oxide to zinc oxide, the temperature of dehydrogenation, and the molar ratio of hydrogen gas to 1,4-butylene glycol are all shown in Table 2. The results of Table 2 are as follows.

Table 2

| Example | 1 | 2 | 3 | 4 | 5 | 6 | 7 |
|---|---|---|---|---|---|---|---|
| Ratio by Weight of Cupric Oxide to Zinc Oxide | 3/1 | 5/1 | ½ | 5/1 | 3/1 | 5/1 | 3/1 |
| Reaction Temperature (°C) | 210 | 210 | 210 | 210 | 210 | 230 | 230 |
| Reaction Pressure (atm) ($10^5$Pa) | 1 | 1 | 1 | 1 | 1 | 1 | 1 |
| Gas Hourly Space Velocity of BOD ($hr^{-1}$) | 4500 | 4500 | 4500 | 4500 | 4500 | 4500 | 4500 |
| Molar Ratio of Hydrogen Gas to BOD | 5/1 | 5/1 | 5/1 | 2/1 | 2/1 | 5/1 | 5/1 |
| Conversion of BOD (mol%) | 97.30 | 98.50 | 92.10 | 95.30 | 93.50 | 98.10 | 97.20 |
| Selectivity of γ-Butyrolactone (mol%) | 96.10 | 97.40 | 90.50 | 97.10 | 96.50 | 91.50 | 90.30 |
| Yield of γ-Butyrolactone (mol%) | 93.50 | 95.94 | 83.35 | 92.53 | 90.22 | 89.76 | 87.77 |

## Example 8

The catalyst precursor prepared by Example 1 was immersed in 1.5 wt% of aqueous barium hydroxide solution. Then, this catalyst was moved to a tubular high-temperature furnace heated to 450°C, and calcined for 4 hours. The component of this catalyst was 55 wt% of copper oxide, 22 wt% of zinc oxide and 1.2 wt% of barium oxide.
The same steps as in Reference Example 1 were carried out with this catalyst. The results are shown in Table 3.

## Example 9

The catalyst precursor prepared by Example 1 was immersed in 1.0 wt% of aqueous calcium hydroxide solution. Then, this catalyst was moved to a tubular high-temperature furnace heated to 450°C, and calcined for 4 hours. The component of this catalyst was 53 wt% of copper oxide, 24 wt% of zinc oxide and 0.81 wt% of calcium oxide.

The same steps as in Reference Example 1 were carried out with this catalyst. The results are shown in Table 3.

## Example 10

The catalyst precursor prepared by Example 1 was immersed in1.0 wt% of aqueous magnesium hydroxide solution. Then, this catalyst was moved to a tubular high-temperature furnace heated to 450°C, and calcined for 4 hours. The component of this catalyst was 49 wt% of copper oxide, 26 wt% of zinc oxide and 0.52 wt% of magnesium oxide.

The same steps as in Reference Example 1 were carried out with this catalyst. The results are shown in Table 3.

## Example 11

The catalyst precursor prepared by Example 1 was immersed in 1.5 wt% of aqueous barium hydroxide solution and 0.3 wt% of aqueous calcium hydroxide solution. Then, this catalyst was moved to a tubular high-temperature furnace heated to 450°C, and calcined for 4 hours. The component of this catalyst was 55 wt% of copper oxide, 22 wt% of zinc oxide, 1.2 wt% of barium oxide and 0.14 wt% of calcium oxide.

The same steps as in Reference Example 1 were carried out with this catalyst. The results are shown in Table 3.

## Example 12

The catalyst precursor prepared by Example 1 was immersed in 1.0 wt% of aqueous calcium hydroxide solution and 0.4 wt% of aqueous magnesium hydroxide solution. Then, this catalystwas moved to a tubular high-temperature furnace heated to 450°C, and calcined for 4 hours. The component of this catalyst was 53 wt% of copper oxide, 24 wt% of zinc oxide, 0.81 wt% of calcium oxide and 0.16 wt% of magnesium oxide.

The same steps as in Reference Example 1 were carried out with this catalyst. The results are shown in Table 3.

## Example 13

The catalyst precursor prepared by Example 1 was immersed in 1.0 wt% of aqueous magnesium hydroxide solution and 0.2 wt% of aqueous barium hydroxide solution. Then, this catalyst was moved to a tubular high-temperature furnace ,heated to 450°C, and calcined for 4 hours. The component of this catalyst was 49 wt% of copper oxide, 26 wt% of zinc oxide, 0.52 wt% of magnesium oxide and 0.11 wt% of barium oxide.

The same steps as in Reference Example 1 were carried out with this catalyst. The results are shown in Table 3.

Table 3

| Example | 8 | 9 | 10 | 11 | 12 | 13 |
|---|---|---|---|---|---|---|
| Reaction Temperature (°C) | 210 | 210 | 210 | 210 | 210 | 210 |
| Reaction Pressure (atm) ($10^5$Pa) | 1 | 1 | 1 | 1 | 1 | 1 |
| Gas Hourly Space Velocity of BOD ($hr^{-1}$) | 4500 | 4500 | 4500 | 4500 | 4500 | 4500 |
| Molar Ratio of Hydrogen Gas to BOD | 5/1 | 5/1 | 5/1 | 5/1 | 5/1 | 5/1 |
| Conversion of BOD (mol%) | 99.50 | 99.20 | 99.60 | 99.90 | 99.50 | 99.80 |
| Selectivity of γ-Butyrolactone (mol%) | 99.10 | 98.60 | 98.10 | 99.80 | 99.10 | 99.30 |
| Yield of γ-Butyrolactone (mol%) | 98.60 | 97.81 | 97.70 | 99.70 | 98.60 | 99.10 |

**Claims**

1. A method for preparing a lactone, which comprises a dehydrocyclization reaction of a diol under a gas phase in the presence of an activated catalyst in which the catalyst comprises a cupric compound, a zinc compound and at least one alkaline earth metal compound on a support, and is reduced to activate at a temperature ranging from 180 to 250°C for 6 to 20 hours with hydrogen gas.

2.  A method according to claim 1, wherein the material of said support is selected from a group consisting of silica, alumina, and their mixture.

3.  A method according to claim 1 or claim 2 wherein said cupric compound is selected from copper (II) nitrate, copper (II) carbonate, copper (II) acetate, copper (II) chloride, copper (II) hydroxide, copper (II) phosphate and copper (II) sulfate.

4.  A method according to any preceding claim, wherein said zinc compound is selected from zinc nitrate, zinc carbonate, zinc acetate, zinc chloride, zinc hydroxide and zinc sulfate.

5.  A method according to any preceding claim, wherein said alkaline earth metal compound is selected from carbonates, hydroxides, silicates and phosphates of beryllium, magnesium, calcium, strontium and barium.

6.  A method according to any preceding claim, wherein the ratio of cupric compound to zinc compound is in the range 6: 1 to 1 : 2 by weight in terms of copper (II) oxide and zinc oxide.

7.  A method according to any preceding claim, wherein the amount of alkaline earth metal compound is in the range 0.01 to 10.wt% based on the total weight of copper (II) oxide and zinc oxide in terms of oxides, when the alkaline earth metal compound is one compound comprising magnesium, calcium or barium.

8.  A method according to any of claims 1 to 6, wherein the amount of alkaline earth metal compound is 0.5 to 20 wt% based on the total weight of copper (II) oxide and zinc oxide in terms of oxides, when the alkaline earth metal compounds comprising two compounds comprising magnesium, calcium or barium.

9.  A method according to claim 1, wherein said lactone is selected from a group consisting of β-propiolactone, β-butyrolactone, γ-butyrolactone, γ-valerolactone, δ-butyrolactone, γ-caprolactone, ε-caprolactone, δ-hydroxyoctylic acid lactone, δ-hydroxynonylic acid lactone, γ- hydroxydecylic acid lactone and δ-hydroxydecylic acid lactone.

10.  A method according to claim 1, wherein said diol is selected from a group consisting of 1,3-propylene glycol, 2-methyl-1,3-propylene glycol, 1,3-butylene glycol, 1,4-butylene glycol, 1,5-pentanediol, 1,4-pentanediol, 1,5-1,5-hexanediol, 1,6-hexanediol, 1,7-heptanediol, 1,8-octanediol, 1,9-nonanediol and 1,10-decanediol,

11.  A method according to claim 10, wherein the diol is 1,4-butylene glycol.

12.  A method according to claim 11, wherein said dehydrocyclization reaction is carried out in a molar ratio of hydrogen gas to 1,4-butylene glycol ranging from (12 to 1): 1.

13.  A method according to claim 11, wherein said dehydrocyclization reaction is carried out with the gas hourly space velocity of 1,4-butylene glycol ranging from 10 to 20,000 hr$^{-1}$.

14.  A method according to claim 1, wherein said dehydrocyclization reaction is carried out at a temperature ranging from 160 to 280°C.

15.  A method according to claim 1, wherein the catalyst bed used for said dehydrocyclization reaction is a fixed bed.

16.  A method according to claim 1, wherein the catalyst bed used for said dehydrocyclization reaction is a fluid bed.

**Patentansprüche**

1.  Verfahren zur Herstellung von Lacton, welches eine Dehydrozyklisierungsreaktion eines Diols unter einer Gasphase in Gegenwart eines aktivierten Katalysators umfasst, wobei der Katalysator eine Kupferverbindung, eine Zinkverbindung und wenigstens eine Erdalkalimetallverbindung auf einem Träger umfasst und zur Aktivierung bei einer Temperatur von 180 bis 250°C für 6 bis 20 Stunden mit Wasserstoffgas reduziert wird.

**2.** Verfahren gemäß Anspruch 1, wobei es sich bei dem Trägermaterial um Siliciumoxid, Aluminiumoxid oder Mischungen davon handelt.

**3.** Verfahren gemäß Anspruch 1 oder Anspruch 2, wobei es sich bei der Kupferverbindung um Kupfer(II)nitrat, Kupfer (II) carbonat, Kupfer(II)acetat, Kupfer(II)Chlorid, Kupfer(II)hydroxid, Kupfer(II)phosphat und/oder Kupfer(II)sulfat handelt.

**4.** Verfahren gemäß wenigstens einem der vorhergehenden Ansprüche, wobei es sich bei der Zinkverbindung um Zinknitrat, Zinkcarbonat, Zinkacetat, Zinkchlorid, Zinkhydroxid und/oder Zinksulfat handelt.

**5.** Verfahren gemäß wenigstens einem der vorhergehenden Ansprüche, wobei es sich bei der Erdalkalimetallverbindung um Carbonate, Hydroxide, Silicate und/oder Phosphate von Beryllium, Magnesium, Calcium, Strontium und/oder Barium handelt.

**6.** Verfahren gemäß wenigstens einem der vorhergehenden Ansprüche, wobei das Gewichtsverhältnis der Kupferverbindung zu der Zinkverbindung im Bereich von 6:1 bis 1:2 bei Kupfer(II)oxid und Zinkoxid beträgt.

**7.** Verfahren gemäß wenigstens einem der vorhergehenden Ansprüche, wobei die Menge der Erdalkalimetallverbindung im Bereich von 0,01 bis 10 Gew.-%, bezogen auf das Gesamtgewicht von Kupfer(II)oxid und Zinkoxid als Oxide, beträgt, wenn die Erdalkalimetallverbindung Magnesium, Calcium oder Barium umfasst.

**8.** Verfahren gemäß wenigstens einem der Ansprüche 1 bis 6, wobei die Menge der Erdalkalimetallverbindung 0,5 bis 20 Gew.-%, bezogen auf das Gesamtgewicht von Kupfer(II)oxid und Zinkoxid als Oxide, beträgt, wenn die Erdalkalimetallverbindungen zwei der Verbindungen Magnesium, Calcium oder Barium umfassen.

**9.** Verfahren gemäß Anspruch 1, wobei es sich bei dem Lacton um β-Propiolacton, β-Butyrolacton, γ-Butyrolacton, γ-Valerolacton, δ-Butyrolacton, γ-Caprolacton, ε-Caprolacton, δ-Hydroxyoctylsäurelacton, δ-Hydroxynonylsäurelacton, γ-Hydroxydecylsäurelacton und/oder δ-Hydroxydecylsäurelacton handelt.

**10.** Verfahren gemäß Anspruch 1, wobei es sich bei dem Diol um 1,3-Propylenglykol, 2-Methyl-1,3-propylenglykol, 1,3-Butylenglykol, 1,4-Butylenglykol, 1,5-Pentandiol, 1,4-Pentandiol, 1,5-Hexandiol, 1,6-Hexandiol, 1,7-Heptandiol, 1,8-Octandiol, 1,9-Nonandiol und/oder 1,10-Decandiol handelt.

**11.** Verfahren gemäß Anspruch 10, wobei es sich bei dem Diol um 1,4-Butylenglykol handelt.

**12.** Verfahren gemäß Anspruch 11, wobei die Dehydrozyklisierungsreaktion bei einem molaren Verhältnis des Wasserstoffgases zu dem 1,4-Butylenglykol im Bereich von (12 bis 1):1 durchgeführt wird.

**13.** Verfahren gemäß Anspruch 11, wobei die Dehydrozyklisierungsreaktion mit der Gasstundenraumgeschwindigkeit von 1,4-Butylenglykol im Bereich von 10 bis 20.000 Std.$^{-1}$ durchgeführt wird.

**14.** Verfahren gemäß Anspruch 1, wobei die Dehydrozyklisierungsreaktion bei einer Temperatur im Bereich von 160 bis 280°C durchgeführt wird.

**15.** Verfahren gemäß Anspruch 1, wobei das für die Dehydrozyklisierungsreaktioon verwendete Katalysatorbett ein Festbett ist.

**16.** Verfahren gemäß Anspruch 1, wobei das für die Dehydrozyklisierungsreaktion verwendete Katalysatorbett ein Flüssigbett ist.

**Revendications**

**1.** Procédé de préparation d'une lactone, qui comprend une réaction de déshydrocyclisation d'un diol sous une phase gazeuse en présence d'un catalyseur activé dans lequel le catalyseur comprend un composé cuprique, un composé du zinc et au moins un composé de métal alcalino-terreux sur un support, et est réduit pour activer à une température allant de 180 à 250°C pendant 6 à 20 heures avec de l'hydrogène gazeux.

**2.** Procédé selon la revendication 1, dans lequel le matériau dudit support est choisi dans un groupe. comprenant la silice, l'alumine et leur mélange.

**3.** Procédé selon la revendication 1 ou 2, dans lequel ledit composé cuprique est choisi parmi le nitrate de cuivre (II), le carbonate de cuivre (II), l'acétate de cuivre (II), le chlorure de cuivre (II), l'hydroxyde de cuivre (II), le phosphate de cuivre (II) et le sulfate de cuivre (II).

**4.** Procédé selon l'une quelconque des revendications précédentes, dans lequel ledit composé du zinc est choisi parmi le nitrate de zinc, le carbonate de zinc, l'acétate de zinc, le chlorure de zinc, l'hydroxyde de zinc et le sulfate de zinc.

**5.** Procédé selon l'une quelconque des revendications précédentes, dans lequel ledit composé de métal alcalino-terreux est choisi parmi des carbonates, des hydroxydes, des silicates et des phosphates de béryllium de magnésium, de strontium et de baryum.

**6.** Procédé selon l'une quelconque des revendications précédentes, dans lequel le rapport du composé cuprique au composé du zinc est dans la plage de 6:1 à 1:2 en poids par rapport à de l'oxyde de cuivre (II) et de l'oxyde de zinc:

**7.** Procédé selon l'une quelconque des revendications précédentes, dans lequel la quantité de composé de métal alcalino-terreux est dans la plage de 0,01 à 10% en poids basé sur le poids total d'oxyde de cuivre (II) et d'oxyde de zinc par rapport aux oxydes, lorsque le composé de métal alcalino-terreux est un composé comprenant du magnésium, du calcium ou du baryum.

**8.** Procédé selon l'une quelconque des revendications 1 à 6, dans lequel dans lequel la quantité de composé de métal alcalino-terreux est de 0,5 à 20% en poids basé sur le poids total d'oxyde de cuivre (II) et d'oxyde de zinc par rapport aux oxydes, lorsque les composés de métal alcalino-terreux comprennent deux composés comprenant du magnésium, du calcium ou du baryum.

**9.** Procédé selon la revendication 1, dans lequel ladite lactone est choisi dans un groupe comprenant la β-propiolactone, la β-butyrolactone, la γ-butyrolactone, la γ-valérolactone, la δ-butyrolactone, la γ-caprolactone, la ε-caprolactone, la lactone de l'acide δ-hydroxyoctylique, la lactone de l'acide δ-hydroxynonylique, la lactone de l'acide γ-hydroxydécyclique et la lactone de l'acide δ-hydroxydécylique.

**10.** Procédé selon la revendication 1, dans lequel ledit diol est choisi dans un groupe comprenant le 1,3-propylèneglycol, le 2-méthyl-1,3-propylèneglycol, le 1,3-butylèneglycol, le 1,4-butylèneglycol, le 1,5-pentanediol, le 1,4-pentanediol, le 1,5-hexanediol, le 1,6-hexanediol, le 1,7-heptanediol, le 1,8-octanediol, le 1,9-nonanediol et le 1,10-décanediol.

**11.** Procédé selon la revendication 10, dans lequel le diol est le 1,4-butylèneglycol.

**12.** Procédé selon la revendication 11, dans lequel ladite réaction de déshydrocyclisation est effectuée dans un rapport molaire d'hydrogène gazeux au 1,4-butylèneglycol allant de (12 à 1):1.

**13.** Procédé selon la revendication 11, dans lequel ladite réaction de déshydrocyclisation est effectuée avec la vitesse spatiale en gaz de 1,4-butylèneglycol de 10 à 20 000 hr$^{-1}$.

**14.** Procédé selon la revendication 1, dans lequel ladite réaction de déshydrocyclisation est effectuée à une température allant de 160 à 280°C.

**15.** Procédé selon la revendication 1, dans lequel ledit lit de catalyseur utilisé pour ladite réaction de déshydrocyclisation est un lit fixe.

**16.** Procédé selon la revendication 1, dans lequel le lit de catalyseur utilisé pour ladite réaction de déshydrocyclisation est un lit fluide.